(19)

Europäisches
Patentamt
European
Patent Office
Office européen
des brevets

(11) **EP 3 967 237 A1**

(12) **EUROPEAN PATENT APPLICATION**

(43) Date of publication:
**16.03.2022  Bulletin 2022/11**

(51) International Patent Classification (IPC):
**A61B 8/06** *(2006.01)*      **A61B 8/08** *(2006.01)*
**G01S 15/89** *(2006.01)*

(21) Application number: **20196161.2**

(22) Date of filing: **15.09.2020**

(52) Cooperative Patent Classification (CPC):
**A61B 8/06; A61B 8/488; A61B 8/5269;**
**G01S 7/5205; G01S 7/52077; G01S 15/8979**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **PALANISAMY, Krishnamoorthy**
  **5656 AE Eindhoven (NL)**

• **PANDYA, Maulik Yogeshbhai**
  **5656 AE Eindhoven (NL)**
• **MANZARI, Sabina**
  **5656 AE Eindhoven (NL)**
• **SETHURAMAN, Shriram**
  **5656 AE Eindhoven (NL)**

(74) Representative: **Philips Intellectual Property &**
**Standards**
**High Tech Campus 52**
**5656 AG Eindhoven (NL)**

(54) **ULTRASOUND DATA PROCESSOR**

(57) An ultrasound data processor for assessing reliability of ultrasound data for determining blood flow values for a patient. The processor is adapted to apply at least a spectral analysis procedure and a wavelet decomposition procedure to input ultrasound data (or data or signals derived therefrom), and to feed the output(s) of said procedures to a classifier algorithm configured to generate a reliability indicator based on these input information. Both spectral analysis and wavelet decomposition are signal analysis techniques which assess periodic properties of data and signals, this being particularly suited to detecting background noise artefacts (such as those generated by electrical knives used in surgery) which do not change the morphology or shape of the signals but do impose a background distortion.

**Beat-to-Beat Common Carotid Flow (cCO CCA)**

Clinically Significant Flow Values with Variations due to Irregular Heart Beats

**FIG. 2**

EP 3 967 237 A1

**Description**

FIELD OF THE INVENTION

The present invention relates to an ultrasound data processor, in particular for analyzing reliability of acquired ultrasound data for deriving hemodynamic measurements.

BACKGROUND OF THE INVENTION

Ultrasound can be used for sensing and monitoring various hemodynamic parameters of a patient, such as blood flow. For example, various blood flow parameters can be sensed through acquiring Pulse Wave Doppler (PWD) ultrasound data and/or B-mode echo data. The ultrasound-derived waveforms may be presented on a display of a patient monitor. The measurements can be acquired continuously, as part of continuous monitoring in some applications.

One important parameter which may be measured is variation or trend in the blood flow values over the time. In general, observable variations in the blood flow values (or any other hemodynamic parameter) may occur either due to clinical factors (such as a change in the patient hemodynamic condition, or irregular heartbeats of the patient) or due to artifacts in the ultrasound data leading to inaccurate hemodynamic parameter calculation.

Ultrasound signals are known to be prone to artifacts, such as those created by external environments, motion and beam steering, which reduce the reliability of the derived waveforms and parameters. The presence of external artefacts affects the accuracy of the flow measurements, and thus the reliability of the trend in flow measurements.

In the case, by way of example, of perioperative care, this care encompasses all phases of a surgical procedure. It typically takes place in surgical centers attached to hospitals. One of the most common and un-avoidable external artifacts in ultrasound based measurements in the perioperative environment are those introduced by electric knives (or electro-knives) used by surgeons during surgical operations. An example of an electro-knife is an electric scalpel. Surgical electric knives use electrical current or radiation to cut, coagulate, desiccate or fulgurate tissue. They are used frequently during surgical operations as they can help prevent blood loss when cutting.

It would be desirable to have means for reliably distinguishing between variations in ultrasound-based hemodynamic parameter measurements caused by clinically relevant factors (e.g. hemodynamic condition, irregular heartbeat), and those due to signal artefacts (e.g. electro-knife noise in ultrasound data).

SUMMARY OF THE INVENTION

The invention is defined by the claims.

According to examples in accordance with an aspect of the invention, there is provided an ultrasound data processor,

the processor comprising an input/output for receiving ultrasound data as an input, the ultrasound data including at least pulse wave Doppler (PWD) ultrasound data,

the processor configured to:

apply a first analysis procedure to the ultrasound data, or a derivative thereof, to generate a first set of one or more analysis parameters, the first analysis procedure comprising a Fourier spectral analysis of the data;
apply a second analysis procedure to the ultrasound data, or a derivative thereof, to generate a second set of one or more analysis parameters, the second analysis procedure comprising wavelet decomposition,
input the first and second sets of analysis parameters to a classifier, wherein the classifier is configured to generate, based on the input analysis parameters, at least one reliability classification for the input ultrasound data indicative of reliability of at least a portion of the input data for determining patient blood flow measurements using the data.

As discussed above, an aim of embodiments of the present invention is to enable determination of background noise artefacts in the data (such as those caused by electro-knife interference). These degrade the quality of the ultrasound signal but do not necessarily change the morphology or general shape of the signal. Embodiments of the invention propose use of spectral analysis (e.g. Fourier spectral analysis) and wavelet analysis, both of which allow analysis of periodic properties of the signal. These techniques are most suited to detecting background distortions in the signal, as opposed to artefacts which cause morphological features of the waveform, and thus enable the relevant artefacts to be identified.

In particular, both Fourier analysis and Wavelet analysis techniques analyze periodic patterns in the signals or periodic characteristics of the data. The background artefacts of interest tend not to change a shape of the acquired signals. However, the inventors have realized that the background artefacts are detectable based on assessment of variations in the periodic properties or patterns of the signals.

The first and second analysis procedures may be applied to the input ultrasound data itself, or to data or signals derived from the input ultrasound data. For example, the input PWD data may be a PWD signal, and from this a PWD spectrogram may be determined. This may be provided as an input to the first and second analysis procedures. In further examples, an envelope of the PWD spectrogram may be extracted, the envelope for example being representative of a time series of maximum Doppler frequency or velocity detected at each time point, and this envelope signal provided as the input to the first and second analysis procedures. These represent non-limiting examples only.

The classifier may be a classifier algorithm. The classifier may be a machine learning algorithm, e.g. a decision tree classifier.

The reliability classification is determined based on the outputs of the first and second analysis procedures. These analysis procedures both analyze periodic patterns of the ultrasound data, or data derived therefrom. Based on this, the classifier is configured, e.g. trained, to derive a classification as to the reliability of the ultrasound data for calculating the blood flow measurements. It may give an indication therefore as to whether variations observable in blood flow values calculated using the ultrasound data are due to clinically relevant factors or clinically non-relevant factors. The classification may be quantitative in some examples (e.g. a reliability score) or may be qualitative (e.g. "reliable" / "not reliable").

The processor may be configured to generate a data output at the input/output indicative of the reliability classification. This may be communicated or transmitted to an external device in some examples.

According to advantageous embodiments, the processor may be configured to receive and process the input ultrasound data in real time. In other words, the reliability classification(s) may be generated in real time with ultrasound data acquisition. This allows the results to be presented in real time to a user, enabling flow measurements to be interpreted in the context of their determined reliability.

According to one or more embodiments, the processor may be further configured to process the input PWD ultrasound data to derive one or more blood flow measurements. Thus in this embodiment, the same processor is used to determine the hemodynamic parameter as is used to assess the reliability of the data. This reduces components, and may also allow in some embodiments the two results to be more easily synchronized or otherwise linked or associated with one another. In some examples, blood flow measurements may be derived continuously or recurrently.

According to one or more embodiments, the processor may be configured to determine a reliability of each of the one or more determined blood flow measurements based on the reliability classification(s) for the ultrasound data (based upon which the flow measurement(s) were calculated). If the reliability classification of the ultrasound data is low for example, a reliability of the flow measurements may be determined to be low, and vice versa.

According to one or more embodiments, the input ultrasound data may further include ultrasound echo data, e.g. B-mode ultrasound data, and wherein the processor is configured to apply the first and second analysis procedures to the ultrasound echo data to further determine a reliability classification for the ultrasound echo data.

The ultrasound echo data can be subsequently used for generating ultrasound image data, for instance for display on the patient monitor along with flow measurements derived using the PWD data.

The processor may be configured to compute an overall classification for the ultrasound data based on a combination of the reliability classifications for the PWD data and the echo data.

In accordance with one or more embodiments, the input PWD ultrasound data may be representative of a PWD signal as a function of time, and wherein the processor is configured to break the signal into multiple temporal portions, and to generate a respective reliability classification for each temporal portion.

According to one or more embodiments, the processor may be further configured to process each of the temporal portions of the PWD ultrasound signal to derive a respective blood flow measurement based on each temporal portion.

The processor may be configured to determine a reliability of each of the respective blood flow measurements based on the reliability classification determined for the respective temporal portion.

In accordance with one or more embodiments, the processor may be further configured to apply a data enhancement procedure to ultrasound data for which the classifier determines a low reliability classification. The low reliability classification may be pre-defined, e.g. a low classification may correspond to a pre-defined range of quantitative reliability scores, or a pre-defined set of one or more qualitative reliability classifications.

Optionally, the data enhancement procedure may comprise application of one or more filters.

According to one or more embodiments, the processor may be adapted to derive a PWD spectrogram based on the input PWD data, and wherein the first and second analysis procedures are applied at least to the PWD spectrogram data. The first analysis procedure may comprise deriving a frequency domain representation of the PWD spectrogram in some examples.

Additionally or alternatively, according to one or more embodiments, the processor may be adapted to process the input PWD data to derive a PWD spectrogram based on the input PWD data, and to further extract a PWD envelope signal from the spectrogram, the envelope signal being representative of a time series of maximum Doppler frequency or velocity detected at each time point, and wherein the first and second analysis procedures are applied at least to a representation of the PWD envelope signal. The first analysis procedure may comprise deriving a frequency domain representation of the envelope signal in some examples.

According to one or more embodiments, the first analysis procedure may comprise determining a ratio of a spectral energy of a peak portion of a frequency domain representation of the ultrasound data, or data derived therefrom, with a spectral energy of a central portion of the frequency domain representation. By way of example, a frequency domain representation of the PWD spectrogram or of an envelope of the PWD spectrogram may be used.

According to one or more embodiments, the reliability classification generated by the classifier may include a reliability score for the ultrasound data.

Examples in accordance with a further aspect of the invention provide a system. The system comprises a patient monitor, comprising a display unit and an input/output. The system further comprises an ultrasound data processor in accordance with any example or embodiment outlined above or described below, or in accordance with any claim of this application.

The patient monitor is configured to receive from the data processor the at least one reliability classification generated for the input ultrasound data. It may be further configured to receive an input indicative of one or more blood flow measurements computed based on the same input ultrasound data. It may be configured to generate a display output on the display device representative of the blood flow measurements, and further representative of the reliability classification(s) for the data.

The patient monitor may receive a respective blood flow measurement calculated by the data processor for each of a plurality of temporal portions of the input ultrasound signal (as discussed above).

The patient monitor may be configured to display a trend of the blood flow measurements over time.

The patient monitor may be further configured to display a visual indicator indicative of a respective reliability classification determined by the data processor for each blood flow measurement (as discussed above).

Examples in accordance with a further aspect of the invention provide a further system.

The further system comprises an ultrasound data processor in accordance with any example or embodiment outlined above or described below, or in accordance with any claim of this application.

The system further comprises a wearable patch, the wearable patch comprising an integrated ultrasound sensing device arranged to acquire ultrasound data including PWD ultrasound data.

The input/output of the ultrasound data processor is communicatively coupled to the wearable patch to receive the acquired ultrasound data from the ultrasound sensing device.

Examples in accordance with a further aspect of the invention provide an ultrasound data processing method.

The method comprises receiving input ultrasound data, the ultrasound data including at least pulse wave Doppler (PWD) ultrasound data;

applying a first analysis procedure to the input ultrasound data, or data derived therefrom, to generate a first set of one or more analysis parameters, the first analysis procedure comprising a Fourier spectral analysis of the data;

applying a second analysis procedure to the input ultrasound data, or data derived therefrom, to generate a second set of one or more analysis parameters, the second analysis procedure comprising wavelet decomposition; and

inputting the first and second sets of analysis parameters to a classifier, wherein the classifier is configured to determine, based on the input analysis parameters, at least one reliability classification for the input ultrasound data indicative of reliability of at least a portion of the input ultrasound data for determining patient blood flow measurements.

Examples in accordance with a further aspect of the invention provide a computer program product comprising computer program code, the computer program code being executable on a processor or computer, wherein the code is configured to cause the processor to perform a method in accordance with any example or embodiment outlined above or described below, or in accordance with any claim of this application.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Figs. 1-4 show example sets of PWD ultrasound data and resulting blood flow estimations for different clinical scenarios;
Fig. 5 illustrates an example processor according to one or more embodiments;
Fig. 6 schematically outlines processing steps performed by an example processor according to one or more embodiments;
Fig. 7 illustrates stages of an example spectral analysis procedure applied to ultrasound data;
Fig. 8 illustrates stages of an example wavelet analysis procedure applied to ultrasound data;
Fig. 9 illustrates stages of a further example spectral analysis procedure applied to ultrasound data;

Fig. 10 illustrates stages of a further example wavelet analysis procedure applied to ultrasound data;
Fig. 11 outlines processing steps of an example processor according to one or more embodiments;
Fig. 12 outlines processing steps of a further example processor according to one or more embodiments;
Fig. 13 schematically illustrates a pre-processing procedure for application to ultrasound data in accordance with one or more embodiments;
Fig. 14 schematically depicts an example system according to one or more embodiments; and
Fig. 15 schematically depicts a further example system according to one or more embodiments.

## DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an ultrasound data processor for assessing reliability of ultrasound data for determining blood flow measurements for a patient. The processor is adapted to apply at least a spectral analysis procedure and a wavelet decomposition procedure to input ultrasound data (or data or signals derived therefrom), and to feed the output(s) of said procedures to a classifier algorithm configured to generate a reliability indicator based on this input information. Both spectral analysis and wavelet decomposition are signal analysis procedures which assess periodic properties of data and signals, this being particularly suited to detecting background data artefacts (such as that generated by electrical knives used in surgery) which do not change the morphology or shape of the data signals but do impose a background distortion.

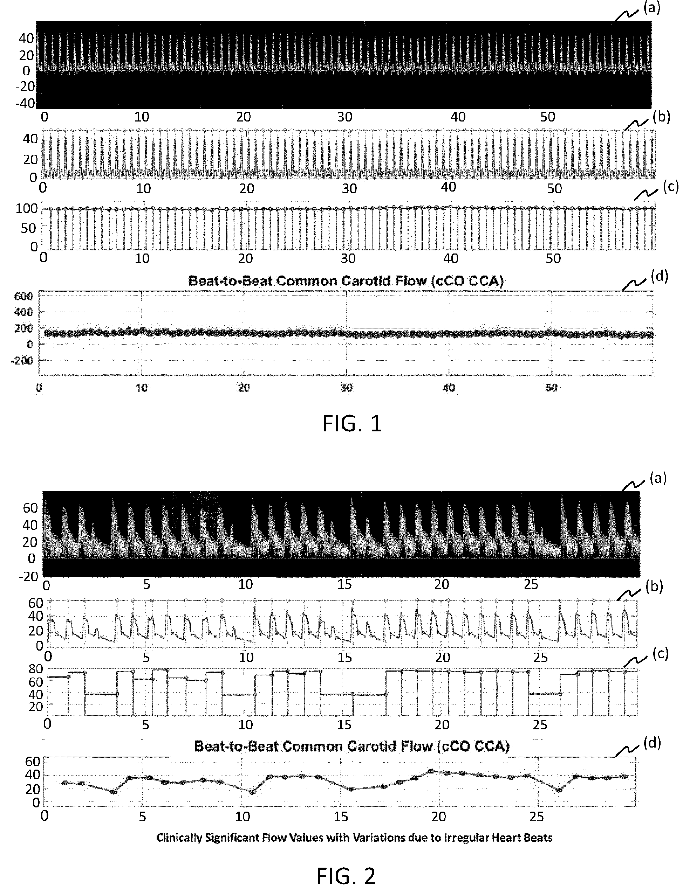

To illustrate the problem which embodiments aim at addressing, Figs. 1-4 show a set of ultrasound signal traces and corresponding clinical parameter traces for a set of different clinical scenarios. For each of Figs. 1-4, graph (a) shows a spectrogram of an acquired pulse wave Doppler (PWD) ultrasound dataset for a patient. Graph (b) shows an envelope of the PWD spectrogram signal trace (a). The envelope traces the maximum detected blood flow Doppler frequency (or velocity) for every time point. It is thus a time-series of maximum frequency (or velocity) at each time-point. The envelope can hence be understood as the "1D time-series waveform of maximum velocity vs time".

Graph (c) shows derived heart rate estimations as function of time, derived based on the PWD trace of graph (b). Graph (d) shows the estimated beat-to-beat blood flow through the carotid artery (y axis; units: ml/min) as a function of time (x-axis; units: seconds) based on the PWD signal trace of graph (b).

Fig. 1 shows the PWD data, and heart rate and blood flow estimations, for a normal (stable) clinical scenario, in which there is no significant background noise in the data and the patient is in a clinically normal state.

Fig. 2 shows the PWD data, and heart rate and blood flow estimations, for a clinical scenario in which the patient is experiencing irregular heartbeats. In this scenario, there is no significant background noise in the ultrasound data (trace (a)). The irregular heartbeats are detectable in the PWD signal trace extracted from the PWD spectrogram (trace (a)), and are shown in heart rate trace (c). The irregular heartbeat leads to detectable irregularity in the blood flow through the carotid artery (trace (d)).

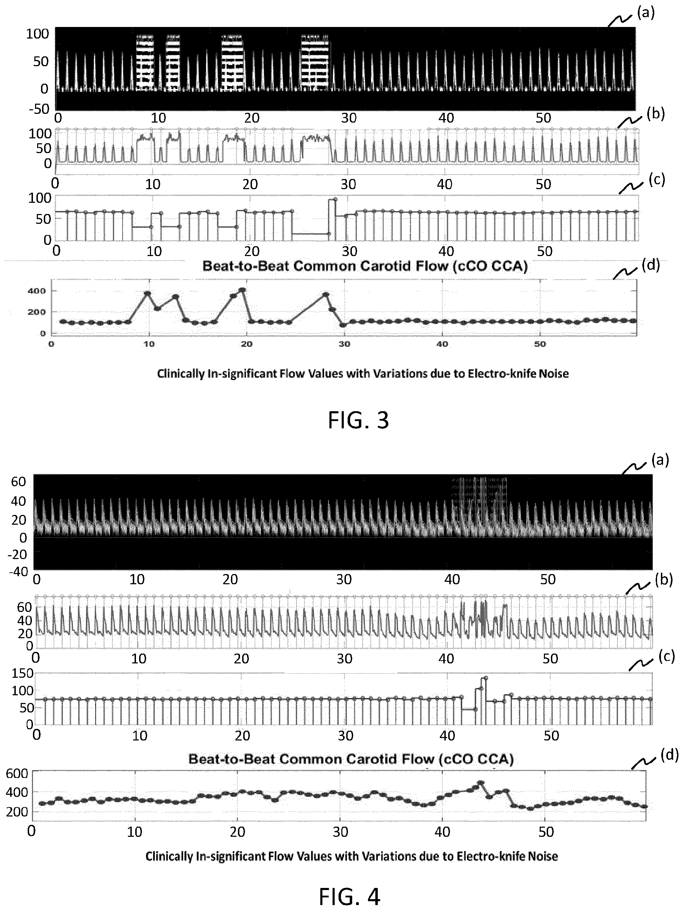

Figs. 3 and 4 show scenarios in which the patient is in a clinically normal hematological state (e.g. no irregular heartbeats), but wherein background artefacts are present in the PWD ultrasound data (graph (a)) due to interference from a surgical electro-knife.

With regards Fig. 3, the background noise is clearly visible in the PWD ultrasound data (graph (a)), as a series of horizontal bars overlaid on the PWD data. The effect of this on the extracted PWD signal trace can be seen in signal trace (b). As shown in signal trace (c), this leads to an erroneous estimation of heart rate values in the portions of the PWD signal trace affected by the background noise artefacts. This consequently results in an estimation of blood flow variation (signal trace (d)) for the patient which erroneously indicates that the patient is experiencing significant irregularity in blood flow (through the carotid artery). A clinician observing the data trends on a patient monitor may thus falsely conclude that the patient in a hematologically abnormal state, leading to potentially unnecessary clinical interventions.

A similar effect can be seen in Fig. 4, which also shows background noise artefacts in the PWD ultrasound data (graph (a)). This leads to distortions in the PWD signal trace extracted from the PWD data (trace (b)), which consequently results in erroneous estimations for the heart rate (trace (c)) in the region of the signal affected by the noise, and erroneous fluctuations in the blood flow estimations (trace (d)).

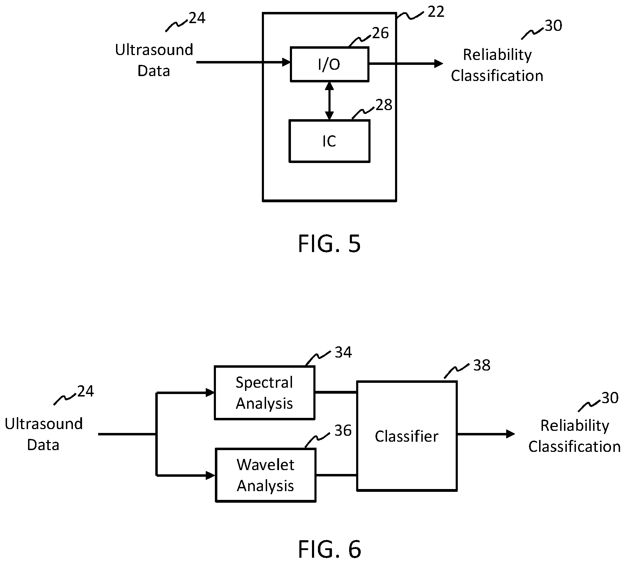

Fig. 5 schematically illustrates components of an example ultrasound data processor according to one or more embodiments of the present invention.

The processor 22 comprises an input/output (I/O) 26 for receiving ultrasound data 24 as an input, the ultrasound data including at least pulse wave Doppler (PWD) ultrasound data. The ultrasound data may be received in real time from an ultrasound transducer unit for example, or it may be received from a datastore in other examples.

In the example of Fig. 5, the data processor 22 further comprises an integrated circuit (IC) 28 which is adapted to perform processing of the ultrasound data received at the I/O 26. The I/O is communicatively coupled with the IC 28.

Fig. 6 schematically illustrates the processing of the input ultrasound data 24 applied by the IC 28 of the processor 22.

The IC 28 of the processor 22 is configured to apply a first analysis procedure 34 to the ultrasound data 24, or data derived therefrom, to generate a first set of one or more analysis parameters. The first analysis procedure comprises a spectral analysis of the data (e.g. Fourier spectral analysis). The first analysis procedure may comprise deriving a frequency-domain representation of the input ultrasound data or data derived therefrom. The output of the first analysis procedure forms a set of one or more first analysis parameters. This will be explained by way of a number of examples further below.

The IC 28 of the processor 22 is further configured to apply a second analysis procedure 36 to the ultrasound data, or data derived therefrom, to generate a second set of one or more analysis parameters. The second analysis procedure comprises wavelet analysis applied to the ultrasound data or data derived therefrom. The output of the wavelet decomposition may provide the second set of one or more analysis parameters. This will be explained by way of a number of examples further below.

The IC is further configured to input the first and second sets of analysis parameters to a classifier 38, wherein the classifier is configured to determine, based on the input analysis parameters, at least one reliability classification 30 for the input ultrasound data indicative of reliability of at least a portion of the input ultrasound data 24 for determining patient blood flow measurements using the data.

The classifier 38 may be a classifier algorithm. It may be stored locally in the processor 22, for example in a memory comprised by the processor, or may be stored external to the processor and retrieved or accessed by the processor. The classifier may be a machine learning algorithm, e.g. a decision tree classifier.

The reliability classification is determined based on the outputs of the first 34 and second 36 analysis procedures. These analysis procedures both analyze periodic patterns of the ultrasound data, or data derived therefrom. Based on this, the classifier 38 is configured or trained to generate a classification 30 as to the reliability of the ultrasound data for calculating the blood flow measurements. It may give an indication therefore as to whether variations observable in blood flow values calculated using the ultrasound data are due to clinically relevant factors or clinically non-relevant factors. The classification may be quantitative in some examples (e.g. a reliability score) or may be qualitative (e.g. reliable / not reliable).

As mentioned, the classifier may be a machine learning algorithm. A machine-learning algorithm is any self-training algorithm that processes input data in order to produce or predict output data. Here, the input data comprises the first and second sets of analysis parameters, and the output data comprises the reliability classification.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. $\pm 1\%$) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

The training input data entries correspond to example first and second analysis parameters. The training output data entries correspond to reliability classifications. The reliability classifications for the training data may for example be determined manually by a specialist, or may for example be determined empirically based on comparison between resulting blood flow values derived from the input ultrasound data for which the analysis parameters were determined and the true blood flow values, for example as measured using a separate blood flow measurement device.

The processor 22 may be configured to generate a data output at the input/output 26 indicative of the reliability classification. This may be communicated or transmitted to an external device in some examples.

The input/output 26 provides a communication interface for example with external devices, such as a patient monitor, or a user interface of a patient monitor.

As discussed above, the first analysis procedure 36 comprises a spectral analysis or decomposition of the ultrasound data. This may comprise applying a Fourier transform to the ultrasound data, or data derived or extracted therefrom, to arrive at a frequency domain representation of the ultrasound data, or data derived therefrom. The analysis parameters may correspond to features or properties of this frequency domain representation, e.g. statistical properties, such as spread of spectral energy. This will be explained in greater detail further below.

As discussed above, the second analysis procedure 34 applied by the processor 22 comprises wavelet analysis. Wavelet decomposition is a well-known signal analysis technique and the skilled person will be aware of a wide variety of means for implementing wavelet analysis.

In the context of embodiments of the present invention, the wavelet decomposition is for the purpose of deriving wavelet coefficients, the absolute and relative values of which may provide parameters which can be used to distinguish reliable data from non-reliable data. In some examples, statistical parameters may be derived related to the wavelet coefficients, which may be used by the classifier 38 to determine the reliability classification.

According to a first set of example embodiments, the processor 22 may be adapted to process the input PWD data 24 to derive a PWD spectrogram based on the input PWD data, and to further extract a PWD envelope signal from the spectrogram, the envelope signal being an envelope of the PWD spectrogram. The first 34 and second 36 analysis procedures may then be applied at least to a representation of the PWD envelope signal.

A PWD spectrogram means a time-frequency representation of the pulse-wave Doppler signal obtained by an ultrasound transducer device at a particular location on the body location. The spectrogram is for example derived from the raw input PWD ultrasound data based on applying a short-time Fourier transform (STFT) to the input data. The spectrogram represents the distribution of Doppler frequencies comprised in the raw PWD signal data over time. It represents the amplitude or magnitude of each Doppler frequency at each time point. Spectrograms are often represented in 2D graphical form, with time represented on the x-axis, frequency on the y-axis and wherein the intensity, color or shading value of each pixel represents the magnitude of the corresponding frequency component at the corresponding time point. Of course, the spectrogram can be represented in other ways, as will be known to the skilled person.

The PWD envelope signal is an envelope of the PWD spectrogram, corresponding to the upper trace of the PWD signal spectrogram (as visible for example in Figs. 1-4, where signal trace (a) in each case shows the acquired PWD signal and signal trace (b) shows the extracted PWD envelope signal in each case).

More particularly, the envelope signal traces the maximum detected blood flow Doppler frequency (or velocity) for every time point. It is thus a time-series of the maximum frequency (or velocity) value at each time-point. The envelope can hence be understood as the "1D time-series waveform of maximum velocity vs time". To extract the envelope signal, an edge-detection algorithm may be used according to one or more examples (e.g. canny edge detection). This permits the upper edge of the PWD spectrogram to be traced, and this trace used for the envelope signal.

One example embodiment which follows the above approach will now be outlined in more detail.

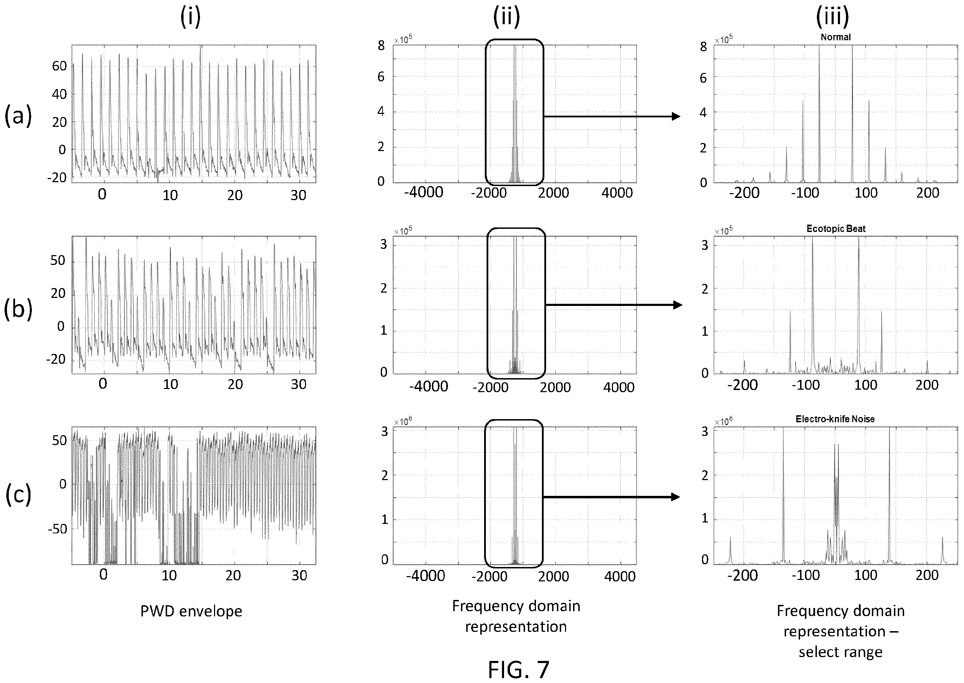

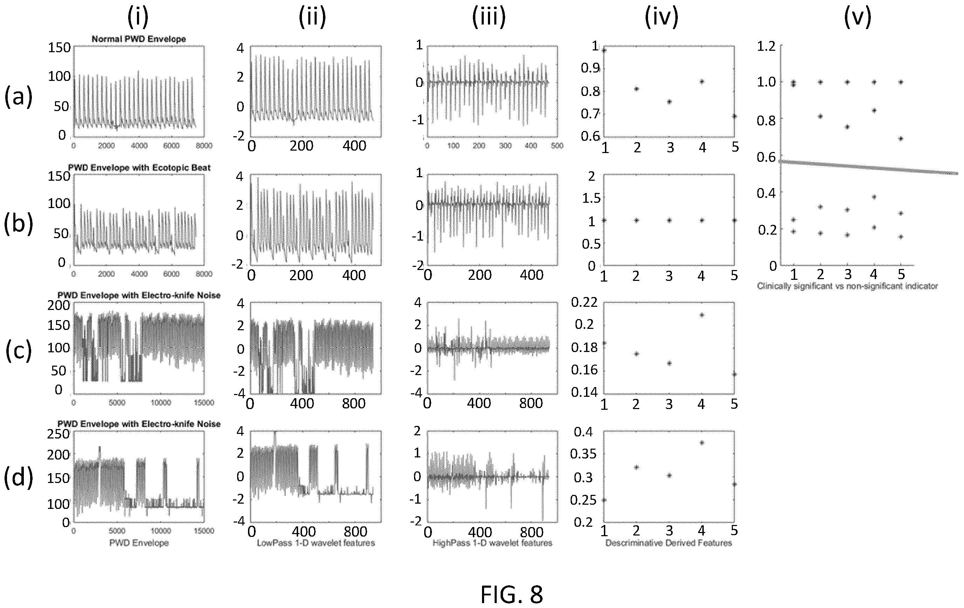

Fig. 7 illustrates processing steps of an example first analysis procedure, applied to an envelope of the PWD spectrogram. Fig. 8 illustrates steps of an example second analysis procedure applied to said PWD envelope signal.

With regards Fig. 7, this shows a set of stages of the data processing applied to the ultrasound data in an example first analysis procedure, for each of three different clinical scenarios. The graphs of row (a) and row (b) correspond to scenarios in which there is no significant background noise in the signal and the PWD data reliably represents the clinical situation for the patient. Row (a) corresponds to a clinically normal patient. Row (b) corresponds to a patient having an irregular heart beat (Ecotropic heart). The graphs of row (c) correspond to a scenario in which there is background noise in the data, by way of example caused by surgical electro-knife interference.

The graphs of column (i) show the PWD envelope signals for each case, corresponding to the extracted envelope of the PWD spectrogram for each case. The y axis corresponds to blood flow velocity in cm/s. The x-axis corresponds to time, in seconds.

The PWD spectrogram for each case may for example be derived by the processor 22 from the (raw) input PWD ultrasound data. Alternatively, the input ultrasound data to the processor may take the form of a PWD spectrogram. The graphs of column (ii) show a frequency domain representation of each of the PWD envelope signals of column (i). This can be derived for example by applying a Fourier transform, e.g. a fast Fourier transform (FFT) to the envelope signal. The y-axis of each of the graphs of column (ii) corresponds to spectral amplitude. The x-axis corresponds to frequency. The frequency x-axis is centered at zero, and the graph includes both positive and negative frequency components (corresponding to positive and negative blood flow velocity components).

The graphs of column (iii) show a select range of each of the frequency domain representations of column (ii), the select range indicated in Fig. 7. This corresponds to a central range or portion of the frequency domain representation in each case. This is presented in Fig. 7 for purposes of ease of illustration and may not be explicitly extracted by the processor during the analysis procedure.

As can be seen in the graphs of column (iii), the spectral energy distribution in the selected region for the different scenarios differs. A common distinction between the spectral distributions for the low background noise data (rows (a) and (b)) and that of the high background noise data (row (c)) is that for the higher noise data, there is concentration of spectral energy near the center of the spectral distribution (i.e. at low frequency components). This results from the fact that background noise artefacts such as those generated by electro-knife interference tend to present in the PWD data at only a select range of (generally low) spectral frequencies, whereas the clinically relevant parts of the PWD data tend to have a more evenly distributed set of frequency components within the PWD data. This feature can be used to identify ultrasound data which may contain background noise artefacts and thus may be less reliable for determination of hemodynamic parameter estimates.

Thus, according to one or more embodiments, the first analysis procedure may comprise determining a ratio of a spectral energy of a peak portion of a frequency domain representation of the PWD ultrasound data, or the PWD envelope signal discussed above, with a spectral energy of a central portion of the frequency domain representation.

According to one example for instance, said ratio, $F_{pc}$, may take the form

$$F_{pc} = \frac{\sum_{n=P+L}^{n=P-L} Xk(n)}{\sum_{n=N+L}^{n=N-L} Xk(n)}$$

where Xk is the frequency domain representation of an envelope of the PWD ultrasound spectrogram, P is the frequency at which a maximum spectral amplitude occurs, $N = N_{fft}/2$ where $N_{fft}$ is the number of frequency points or bins used for computing the frequency domain representation, and L is the number of neighboring data points (around the maximum point P), $n$, used for computation of the spectral energy at said maximum point P. For example L defines the chosen 'half width' (in terms of the number of frequency bins) of the central portion of the frequency distribution and of the peak portion of the frequency distribution. L can be selected as desired in order to capture an appropriate portion of the distribution around the peak frequency point, P, and the center frequency point, N. N may correspond to the Nyquist frequency for example. In some examples, L may be chosen to be less than a certain fraction of N, e.g. less than 1/4 of N, less than 1/6 of N, less than 1/8 of N or less than 1/10 of N for example.

Fig. 8 illustrates the data processing stages of a second analysis procedure comprising wavelet analysis applied to the PWD envelope signal derived from the PWD spectrogram.

Again, the different rows show different clinical scenarios. Rows (a) and (b) correspond to data with no significant background noise, and rows (c) and (d) correspond to scenarios in which the data does contain background noise artefacts, for example caused by electro-knife interference. More particularly, row (a) corresponds to a clinically normal patient. Row (b) corresponds to a patient having irregular heart beat (Ecotropic heart). Rows (c) and (d) correspond to different scenarios in which the data contains background noise artefacts.

The graphs of column (i) show the PWD envelope signals for each case, corresponding to the extracted envelope of the PWD spectrogram for each case (as discussed in more detail above).

The second analysis procedure in this example comprises applying approximation (low pass) and detail (high pass) wavelet filters to the PWD envelope signal to derive approximation $CA_i$ and detail $CD_i$ wavelet coefficients respectively, where i indexes the decomposition level of the coefficient. The wavelet decomposition can be applied with any desired number, N, of decomposition levels. The wavelet coefficients may be continuous wavelet coefficients.

The wavelet coefficients obtained for PWD data which includes background noise artefacts differ from those derived for PWD data without background noise in reliable ways. These differences can be used to identify ultrasound data which may contain background noise artefacts and thus which may be unreliable for deriving blood flow measurements.

By way of example, and with reference to Fig. 8, various different analysis parameters may be derived based on the derived wavelet coefficients for the PWD envelope signal. One or more of these may be fed to the classifier 38 or used in deriving the reliability classifier for the corresponding ultrasound data.

One example analysis parameter may be the ratio of the absolute standard deviation of different wavelet coefficients, for example:

$$\mathrm{WASD} = \frac{DA_4}{DA_1}$$

where,

$$DA_i = \sqrt{\frac{1}{L_i - 1} \sum |CD_i(n) - CD_i(n-1)|^2}$$

wherein $L_i$ is the length of the wavelet coefficient $CD_i$ (e.g. in the time-domain). The wavelet coefficient may be a continuous wavelet coefficient, plotted as a function of time.

A further example analysis parameter may be a ratio of power spectral density:

$$WPSD = \frac{PD_4}{PD_1}$$

where,

$$PD_i = \frac{\sum CD_i^2}{L_i}$$

wherein $L_i$ is the length of the wavelet coefficient $CD_i$ (e.g. along the time axis).

The graphs of column (ii) represent example approximation (i.e. low pass) wavelet coefficients derived from wavelet decomposition of the PWD envelopes in each case. The graphs of column (iii) represent example detail (i.e. high pass) wavelet coefficients derived from wavelet decomposition of the PWD envelopes in each case. The approximation and detail coefficients are each plotted as continuous waveforms, as a function of time. The x-axis in each of the graphs of (ii) and (iii) correspond to time and the y-axis corresponds to wavelet amplitude.

Wavelet decomposition procedures are well known in the art and the skilled person would immediately recognize means for performing the decomposition procedure. More detail on example wavelet decomposition procedures can be found for example in the book: Daubechies, I. Ten Lectures on Wavelets, CBMS-NSF Regional Conference Series in Applied Mathematics. Philadelphia, PA: SIAM Ed, 1992. Details can also be found in the paper: Mallat, S. G. "A Theory for Multiresolution Signal Decomposition: The Wavelet Representation," IEEE Transactions on Pattern Analysis and Machine Intelligence. Vol. 11, Issue 7, July 1989, pp.674-693.

The graphs of column (iv) show example derived WPSD values for each of five different patients, calculated according to the equations set out above. The y-axis corresponds to the WPSD value and the x-axis corresponds to patient number.

The graph of column (v) shows the features of each of the graphs in column (iv) plotted on the same graph. All of the data points above the line shown on the graph are the wavelet features derived from the PWD data without background artefacts (rows (a) and (b)), and all of the data points below the line are features derived from the PWD data with background artefacts (row (c) and (d)). Thus, from this, it can be seen how the features derived and shown in the graphs of column (iv) may be used by a classifier to discriminate between data containing background noise (and therefore unreliable) and data not containing background noise (and therefore reliable).

According to a second set of one or more embodiments, the processor may be adapted to derive a PWD spectrogram based on the input PWD data, and wherein the first and second analysis procedures are applied at least to the PWD spectrogram data. At least the first analysis procedure may comprise deriving a frequency domain representation of the PWD spectrogram.

Thus, in this set of embodiments, the first and second analysis procedures are applied to the PWD spectrogram itself, not to the envelope signal extracted from it. The PWD spectrogram represents the distribution of Doppler frequencies in the input PWD data as a function of time. It represents the amplitude or magnitude of each Doppler frequency at each time point. It thus comprises data which varies over two dimensions (frequency and time). The PWD spectrogram may therefore be referred to herein as the 2D PWD spectrogram.

Spectrograms are often represented in 2D graphical form, with time represented on the x-axis, frequency on the y-axis and wherein the intensity, color or shading value of each pixel represents the magnitude of the relevant frequency component at the relevant time point. Of course, the spectrogram can be represented in other ways, as will be known to the skilled person.

One example embodiment which follows the above-described approach will now be outlined in more detail.

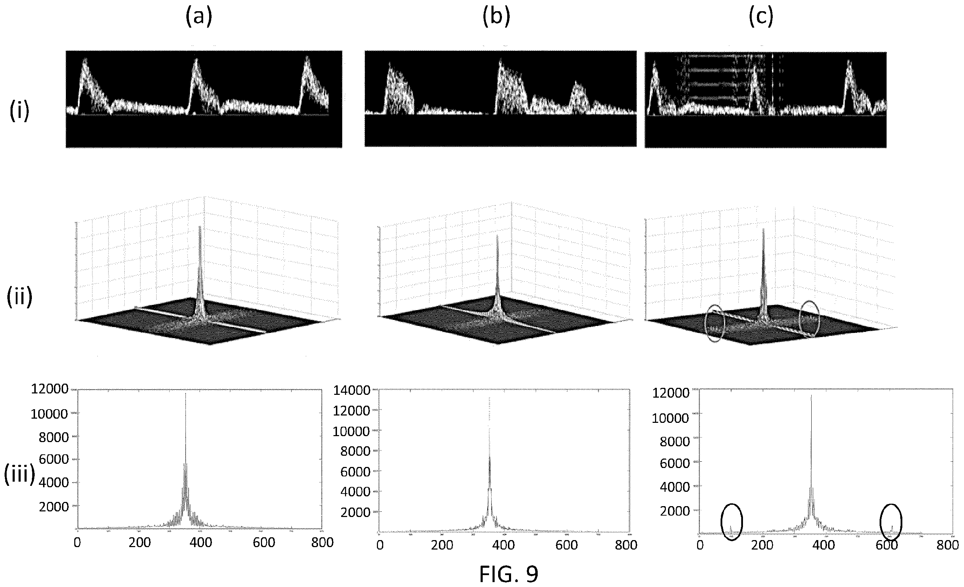

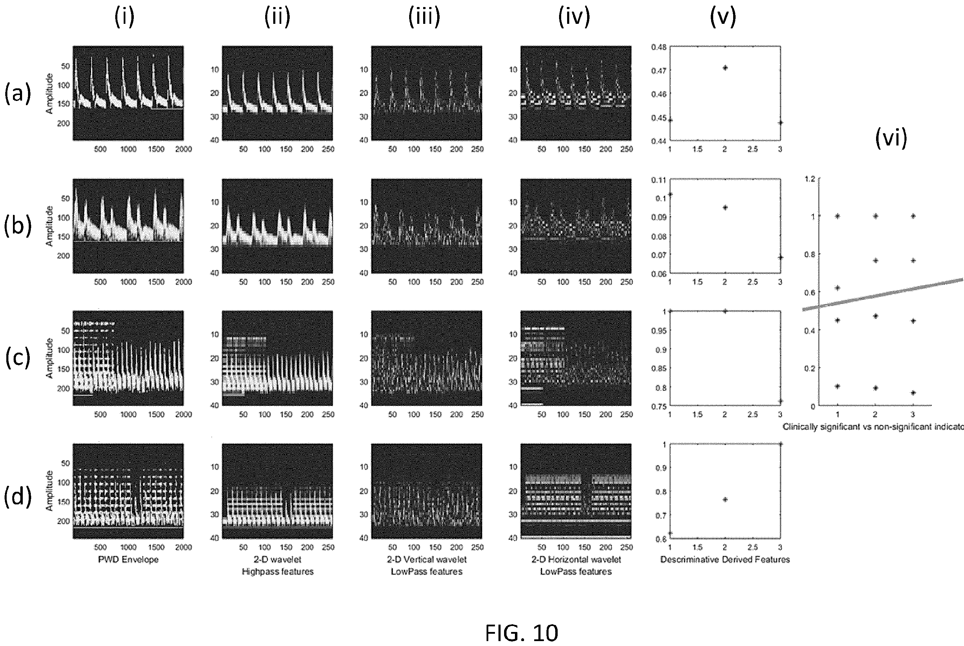

Fig. 9 shows stages of the first analysis procedure applied to the PWD spectrogram. Fig. 10 illustrates the second analysis procedure applied to the PWD spectrogram.

With regards to Fig. 9, this shows a set of stages of the data processing applied to the ultrasound data in an example first analysis procedure, for each of three different clinical scenarios. The graphs of column (a) and column (b) correspond to scenarios in which there is no significant background noise in the signal and the PWD data reliably represents the clinical situation for the patient. Column (a) corresponds to a clinically normal patient. Column (b) corresponds to a patient having irregular heartbeats (Ecotropic heart). The graphs of column (c) correspond to a scenario in which there is background noise in the data, by way of example caused by surgical electro-knife interference.

The figures of row (i) illustrate the PWD spectrogram for each case. The PWD spectrogram for each case may for example be derived by the processor 22 from the (raw) input PWD ultrasound data. Alternatively, the input ultrasound data to the processor may take the form of a PWD spectrogram.

The graphs of row (ii) show a frequency domain representation of each of the PWD spectrograms of row (i). As mentioned above, since the spectrogram covers both the frequency and time dimensions, the frequency domain representation also extends in these two dimensions. The x-axis corresponds to the frequency, the y-axis to time, and the z-axis to amplitude or spectral power. The frequency domain representation can be derived for example by applying a Fourier transform, for example the fast Fourier transform (FFT) to the PWD spectrogram.

The graphs of row (iii) show an average of the frequency spectrum over all of the time frames of the 2D PWD frequency spectrum of row (ii). The x-axis corresponds to frequency and the y-axis corresponds to the frequency amplitude.

The circled areas in the graphs of Fig. 9 (c)(ii) and (c)(iii) indicate spectral components of the data of scenario (c) caused by background noise artefacts in the data, associated for example with electro-knife interference.

As can be seen in the graphs (iii), the spectral energy distribution for the noise-free scenarios (a) and (b) differs from that of the noise-containing scenario (c) in comprising additional spectral energy components away from the central peak (shown in the circled regions). This results from the fact that background noise artefacts, such as those generated by electro-knife interference, tend to present in the PWD data at only a select range of spectral frequencies, whereas the clinically relevant parts of the PWD data tend to have a more evenly distributed set of frequency components within the PWD data. This feature can be used to identify ultrasound data which may contain background noise artefacts and thus may be less reliable for determination of hemodynamic parameter estimates.

According to the present example, the first analysis procedure may comprise: deriving a frequency-domain representation of the 2D PWD spectrogram data (see Fig. 9, row (ii)) and further deriving a time-average of said PWD spectrogram frequency domain representation by averaging over all time frames of the representation (see Fig. 9, row (iii)). The first analysis procedure may further comprise determining a ratio of a spectral energy of a central portion of the time-average frequency domain representation of the PWD spectrogram with a spectral energy of a peak portion.

By way of example, said ratio may take the form:

$$F_{cp} = \frac{\sum_{n=N+L}^{n=N-L} X_{ka}(n)}{\sum_{n=P+L}^{n=P-L} X_{ka}(n)}$$

where $X_{ka}$ is the average of the frequency spectrum of all of the 2D PWD frequency domain representation frames, P is the frequency at which a maximum spectral amplitude occurs in $X_{ka}$, $N = N_{fft}/2$ where $N_{fft}$ is the number of frequency bins used for computing the frequency domain representation, and L is the number of neighboring data points, n, used for computation of spectral energy. For example L defines the chosen 'half width' (in terms of the number of frequency bins) of the central portion of the frequency distribution and of the peak portion of the frequency distribution. L can be selected as desired in order to capture an appropriate portion of the distribution around the peak frequency point, P, and the center frequency point, N. N may correspond to the Nyquist frequency for example. In some examples, L may be chosen to be less than a certain proportion of N, e.g. less than 1/4 of N, less than 1/6 of N, less than 1/8 of N or less than 1/10 of N.

By way of example, the result of this ratio may provide the analysis parameter which is subsequently provided to the classifier algorithm 38 for use in deriving the reliability classification.

Fig. 10 illustrates application of an example second analysis procedure, comprising wavelet decomposition, to the (2D) PWD spectrogram.

Again, the different rows show different clinical scenarios. Rows (a) and (b) correspond to data with no significant background noise, and rows (c) and (d) correspond to scenarios in which the data does contain background noise artefacts, for example caused by electro-knife interference. More particularly, row (a) corresponds to a clinically normal patient. Row (b) corresponds to a patient having irregular heart beat (Ecotropic heart). Rows (c) and (d) correspond to different scenarios in which the data contains background noise artefacts.

The graphs of column (i) show the PWD spectrogram for each case (as discussed in more detail above).

The second analysis procedure in this example comprises applying approximation (low pass), horizontal detail (high pass), vertical detail, and diagonal detail wavelet filters to the PWD spectrogram to derive approximation $CA_i$ and horizontal detail CHi, vertical detail, CVi, and diagonal detail, $CD_i$, wavelet coefficients respectively, where i indexes the decomposition level of the coefficient. This is a 2D Wavelet decomposition process. The wavelet decomposition can be applied with any desired number, N, of decomposition levels. The Wavelet decomposition procedure applied in this case is described in more detail for example in the paper: Mallat, S. G. "A Theory for Multiresolution Signal Decomposition: The Wavelet Representation," IEEE Transactions on Pattern Analysis and Machine Intelligence. Vol. 11, Issue 7, July 1989, pp. 674-693. It is also described in the book: Meyer, Y. Wavelets and Operators. Translated by D. H. Salinger. Cambridge, UK Cambridge University Press, 1995.

For application of the wavelet decomposition, the PWD spectrogram is processed in the form of a 2D image comprising pixels, each with a pixel value representative of amplitude of a given Doppler frequency component at a given time. This approach is well known in the field of signal processing, particularly for example in the field of speech or audio processing, in which speech spectrograms are typically visualized and processed in this way.

As explained above, the wavelet coefficients obtained for PWD data which includes background noise artefacts differ from those derived for PWD data without background noise in reliable ways. These differences can be used to identify ultrasound data which may contain background noise artefacts and thus which may be unreliable for deriving blood flow measurements.

By way of example, and with reference to Fig. 10, various different analysis parameters may be derived based on the derived wavelet coefficients for the PWD envelope signal. One or more of these may be fed to the classifier 38 or use in deriving the reliability classification for the corresponding ultrasound data.

One example analysis parameter may be the wavelet power ratio, WPR, which may be defined as:

$$WPR_i = \frac{\sum CA_1^2}{\sum CH_i^2 + \sum CV_i^2 + \sum CD_i^2}$$

where, i indexes the wavelet decomposition level.

With reference to Fig. 10, the graphs of column (ii) represent example approximation (i.e. low pass) wavelet coefficients derived from wavelet decomposition of the PWD envelopes in each case. The graphs of column (iii) represent example vertical detail (i.e. high-pass) coefficients derived from wavelet decomposition of the PWD envelopes in each case. The graphs of column (iv) represent example horizontal detail coefficients. The wavelet coefficients are continuous coefficients and are plotted as continuous waveforms as a function of time in each of the graphs of column (ii), (iii) and (iv). The y-axis in each case corresponds to the wavelet coefficient amplitude, the x-axis corresponds to time.

The graphs of column (v) show the wavelet power ratios, WPR, calculated for each of the scenarios of rows (a)-(d), to decomposition levels 1, 2, and 3.

The graph of column (v) shows the wavelet power ratios of each of the graphs in column (iv) plotted on the same graph. All of the data points above the line shown on the graph are the WPR results derived from the PWD data without background artefacts (rows (a) and (b)), and all of the data points below the line are WPR results derived from the PWD data with background noise artefacts (row (c) and (d)). Thus, from this, it can be seen how the analysis parameters derived and shown in the graphs of column (iv) may be used by a classifier to discriminate between data containing background noise (and therefore being unreliable) and data not containing background noise (and therefore being reliable).

The wavelet power ratio may provide the analysis parameter output of the second analysis procedure, which is subsequently fed to the classifier algorithm 38 for deriving the reliability classification.

Above have been described two example first and second analysis procedures which may be applied to the input ultrasound data according to one or more embodiments to derive the first and second analysis parameters for input to the classifier algorithm. In the first example, an envelope of a spectrogram of the PWD data is extracted and spectral and wavelet analysis applied to this 1D envelope signal. In the second example, the 2D PWD spectrogram (time-frequency domain) is derived from the input PWD data and spectral and wavelet analysis applied to this.

According to one or more embodiments, the first and second analysis procedures may be applied as described above to both the (2D) PWD spectrogram and to the (1D) PWD envelope extracted from the PWD spectrogram. The analysis coefficients derived from both of these processes may be fed to the classifier 28 for deriving the reliability classification.

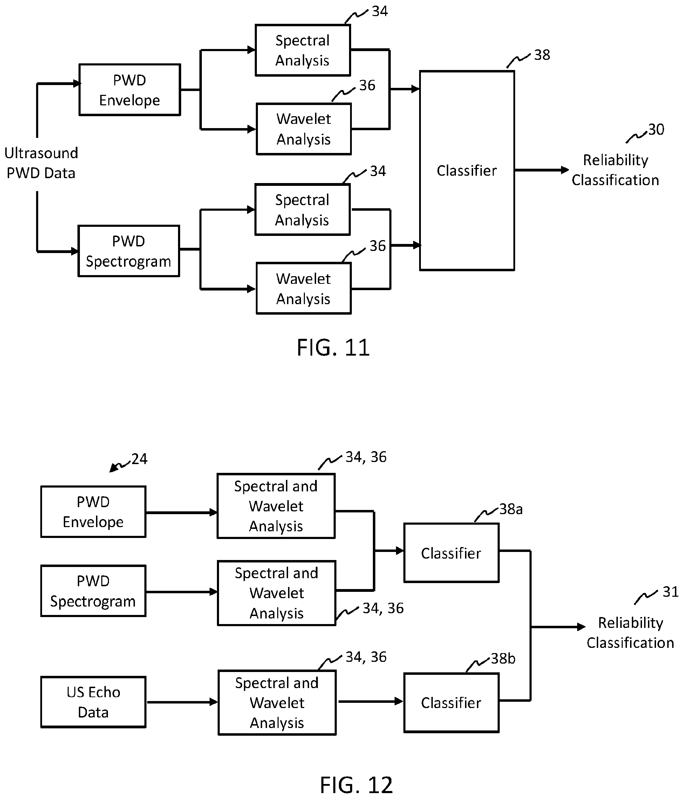

This is illustrated schematically in Fig. 11. From the input PWD ultrasound data, a spectrogram is derived from the PWD data. An envelope of the PWD spectrogram is extracted representative of the time series of maximum frequency or velocity values at each time point. A first analysis procedure comprising spectral analysis 34 and a second analysis procedure comprising wavelet analysis 36 is applied to both the PWD spectrogram and to the extracted PWD envelope signal. First and second sets of analysis parameters are derived in each case, and provided as inputs to the classifier 38. The classifier, based on these inputs, is configured to derive a reliability classification 30, such as a score or grading of the input ultrasound data. The reliability classification indicates reliability of the data for deriving blood flow measurements. The classifier may be a machine learning algorithm such as a decision tree classifier for example.

According to one or more embodiments, the input ultrasound data may further include ultrasound echo data, e.g. B-mode ultrasound data, and wherein the processor is configured to apply the first and second analysis procedures to the ultrasound echo data to further determine a reliability classification for the ultrasound echo data. The ultrasound echo data can be subsequently used for generating ultrasound image data, for instance for display on the patient monitor along with flow measurements derived using the PWD data.

The processor may be configured to compute an overall classification for the ultrasound data based on a combination of the reliability classifications for the PWD data and the echo data.

An example is schematically outlined in Fig. 12. Here, as in the example of Fig. 11, the first 34 and second 36 analysis procedures are applied to both the PWD spectrogram and the PWD envelope signal, and the resulting analysis parameters provided to a first classifier 38a to derive a reliability classification. In addition, ultrasound echo data (e.g. B-mode ultrasound data) is also processed with a first analysis procedure 34 comprising spectral analysis and a second analysis procedure 36 comprising wavelet analysis. The output analysis parameters from these processes are then fed to a second classifier 38b which is configured to derive a reliability classification for the echo data. A combined reliability classification 31 or quality score may further be generated based on a combination of the reliability classifications for the PWD data and for the echo data. By way of example, the overall reliability or quality score may be determined as the maximum of the reliability or quality scores for the PWD and echo data, or the average of the scores for example.

According to one or more embodiments, the processor may be further configured to process the input PWD ultrasound data to derive one or more blood flow measurements. Thus in this embodiment, the same processor is used to determine the hemodynamic parameter as is used to assess the reliability of the data. The blood flow measurements may be derived continuously or recurrently. The processor may additionally or alternatively be configured to determine one or more other hemodynamic parameters based on the input ultrasound data.

Blood flow measurements may be derived from input PWD data based on extracting blood flow velocity, v, from the PWD data and applying the following equation:

$$\text{Blood flow } = vAr$$

where A is arterial cross-sectional area, and r is the heart rate. Further details of a method for deriving blood flow measurements from ultrasound data can be found in the paper: Blanco, P. Volumetric blood flow measurement using Doppler ultrasound: concerns about the technique. J Ultrasound 18, 201-204 (2015).

According to one or more embodiments, the processor 22 may be configured to determine a reliability of each of the one or more blood determined flow measurements based on the reliability classification(s) for the ultrasound data (based upon which the flow measurement(s) were calculated). If the reliability classification of the ultrasound data is low for example, a reliability of the flow measurements may be determined to be low, and vice versa.

In accordance with one or more embodiments, the input PWD ultrasound data may be representative of a PWD signal as a function of time, and wherein the processor is configured to break the signal into multiple temporal portions, and to generate a respective reliability classification for each temporal portion.

According to one or more embodiments, the processor 22 may be further configured to process each of the temporal portions of the PWD ultrasound signal to derive a respective blood flow measurement based on each temporal portion.

The processor 22 may be configured to determine a reliability of each of the respective blood flow measurements based on the reliability classification determined for the respective temporal portion.

In accordance with one or more embodiments, the processor 22 may be further configured to apply a data enhancement procedure to ultrasound data for which the classifier 38 determines a low reliability classification, for example in advance of deriving blood flow measurement values from the data.

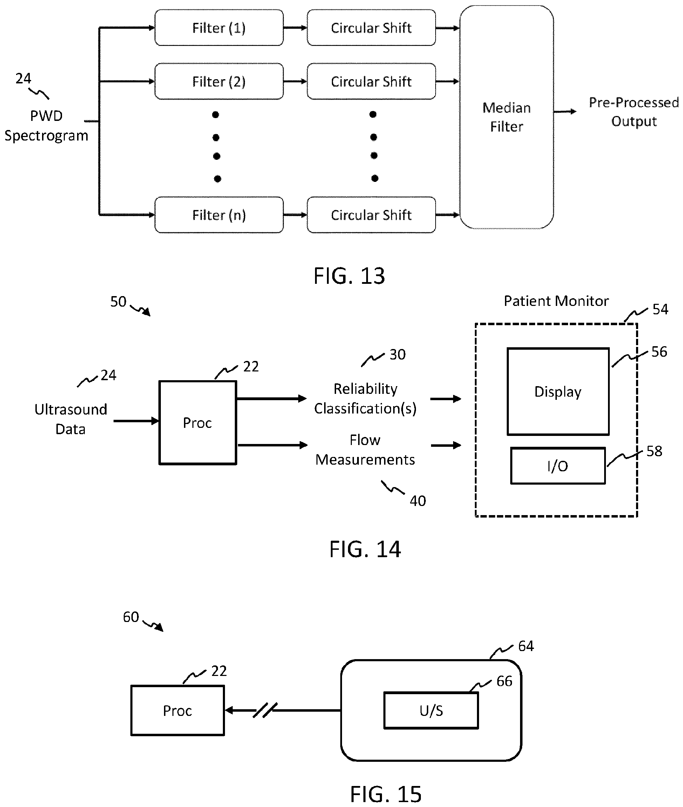

An example is schematically illustrated in Fig. 13. A 2D PWD spectrogram is derived from the input PWD data (as described in more detail above). The 2D PWD spectrogram is then passed in parallel through a plurality, n, of filters. This results in n 2D spectrograms, one for each filter. After each filter, a circular shift is applied to the data. The outputs from the total set of filters is then passed through a median filter which combines the n filtered spectrograms into one PWD filtered spectrogram. The output of this provides filtered (pre-processed) ultrasound data.

For the processing, the spectrogram is treated in the form of a 2D image comprising pixels, each with a pixel value representative of amplitude of a given Doppler frequency component at a given time. This approach is well known in the field of signal processing, particularly for example in the field of speech or audio processing, in which speech spectrograms are typically visualized and processed in this way.

The filters are applied at each pixel level of the spectrogram. It uses the central pixel and the surrounding layer of 8 pixels that lie at most one pixel away in the different directions. By way of one advantageous examples, the following filters may be used: [-1, -1], [-1,0], [-1,1], [0, -1], [0,0], [0,1], [1, -1], [1,0] and [1,1].

According to one or more examples, the classification generated by the classifier for the ultrasound data may comprise a score or grade. Depending upon the score or grade, the data may either be: discarded (in case of a low grade), have a pre-processing data enhancement procedure applied (in case of a medium grade), or be passed without any pre-processing (in case of a high quality grade). In some examples, the filtering process described above may be applied only to data having a reliability classification score which falls within a defined range.

Examples in accordance with a further aspect of the invention provide a patient monitoring system. An example system 50 is schematically outlined in Fig. 14.

The system 50 comprises a patient monitor 54, comprising a display unit 56 and an input/output 58. The system further comprises an ultrasound data processor 22 in accordance with any example or embodiment outlined above or described below, or in accordance with any claim of this application.

The patient monitor 54 is configured to receive from the data processor 22 the at least one reliability classification 30 generated for the input ultrasound data 24. It may be further configured to receive an input indicative of one or more blood flow measurements 40 computed based on the same input ultrasound data 24. It may be configured to generate a display output on the display unit 56 representative of the blood flow measurements, and further representative of the reliability classification(s) for the data.

The patient monitor 54 may receive a respective blood flow measurement 40 calculated by the data processor 22 for each of a plurality of temporal portions of the input ultrasound signal (as discussed above).

The patient monitor 54 may be configured to display a trend of the blood flow measurements over time.

The patient monitor 54 may be further configured to display a visual indicator indicative of a respective reliability classification 30 determined by the data processor 22 for each blood flow measurement 40 (as discussed above).

The input ultrasound data may be received from an ultrasound sensing device, e.g. an ultrasound transducer device. The data may be processed by the processor 22 in real time.

Examples in accordance with a further aspect of the invention may provide a further system.

The system 60 comprises an ultrasound data processor 22 in accordance with any example or embodiment outlined above or described below, or in accordance with any claim of this application.

The system 60 further comprises a wearable patch 64, the wearable patch comprising an integrated ultrasound sensing device 66 arranged to acquire ultrasound data including PWD ultrasound data.

The input/output of the ultrasound data processor 22 is communicatively coupled to the wearable patch 64 to receive the acquired ultrasound data from the ultrasound sensing device. The ultrasound sensing device may comprise an ultrasound transducer array for example.

Although in the example of Fig. 15, a patch-based ultrasound sensing device is provided, in other examples, a different type of ultrasound sensing device may be used, e.g. an ultrasound sensing probe.

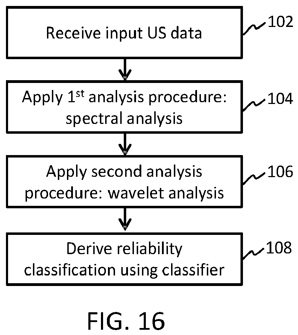

Examples in accordance with a further aspect of the invention provide an ultrasound data processing method. An example method is outlined in block diagram form in Fig. 16.

The method comprises receiving 102 input ultrasound (US) data, the ultrasound data including at least pulse wave Doppler (PWD) ultrasound data.

The method further comprises applying 104 a first analysis procedure to the ultrasound data, or data derived therefrom, to generate a first set of one or more analysis parameters, the first analysis procedure comprising a spectral analysis of the data (e.g. Fourier analysis).

The method further comprises applying 106 a second analysis procedure to the ultrasound data, or data derived therefrom, to generate a second set of one or more analysis parameters, the second analysis procedure wavelet decomposition.

The method further comprises inputting the first and second sets of analysis parameters to a classifier, wherein the classifier is configured to determine 108, based on the input analysis parameters, at least one reliability classification for the input ultrasound data indicative of reliability of at least a portion of the input ultrasound data for determining patient blood flow measurements.

Examples in accordance with a further aspect of the invention also provide a computer program product comprising computer program code, the computer program code being executable on a processor or computer, wherein the code is configured to cause the processor to perform a method in accordance in accordance with any example or embodiment outlined above or described below, or in accordance with any claim of this application.

As discussed above, the system makes use of processor to perform the data processing. The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

**Claims**

1. An ultrasound data processor (22),
   the processor comprising an input/output (26) for receiving ultrasound data (24) as an input, the ultrasound data including at least pulse wave Doppler (PWD) ultrasound data,
   the processor configured to:

   apply a first analysis procedure (34) to the ultrasound data, or data derived therefrom, to generate a first set of one or more analysis parameters, the first analysis procedure comprising a Fourier spectral analysis of the data;
   apply a second analysis procedure (36) to the ultrasound data, or data derived therefrom, to generate a second set of one or more analysis parameters, the second analysis procedure comprising wavelet decomposition,
   input the first and second sets of analysis parameters to a classifier (38), wherein the classifier is configured to determine, based on the input analysis parameters, at least one reliability classification (30) for the input ultrasound data indicative of reliability of at least a portion of the input data for determining patient blood flow measurements using the data.

2. An ultrasound data processor (22) according to claim 1, wherein the processor is configured to generate a data output at the input/output indicative of the reliability classification.

3. An ultrasound data processor (22) according to claim 1 or 2, wherein the processor is configured to receive and process the input ultrasound data in real time.

4. An ultrasound data processor (22) according to any of claims 1-3, wherein the processor is further configured to process the input PWD ultrasound data to derive one or more blood flow measurements.

5. An ultrasound data processor (22) according to claim 4, wherein the processor is configured to determine a reliability of each of the one or more blood flow measurements based on the reliability classification(s) for the ultrasound data.

6. An ultrasound data processor (22) according to any of claims 1-5, wherein the input ultrasound data further includes ultrasound echo data, e.g. B-mode ultrasound data, and wherein the processor is configured to apply the first and second analysis procedures to the echo data to further determine a reliability classification for the echo data;
   and optionally wherein the processor is configured to compute an overall classification for the ultrasound data based on a combination of the reliability classifications for the PWD data and the echo data.

7. An ultrasound data processor (22) according to any of claims 1-6, wherein the input PWD ultrasound data is representative of a PWD signal over time, and wherein the processor is configured to break the signal into multiple temporal portions, and to generate a respective reliability classification for each temporal portion.

8. An ultrasound data processor (22) according to claim 7, wherein the processor is further configured to process each of the temporal portions of the PWD ultrasound signal to derive a respective blood flow measurement based on each temporal portion, and
optionally wherein the processor is configured to determine a reliability of each of the respective blood flow measurements based on the reliability classification determined for the respective temporal portion.

9. An ultrasound data processor (22) as claimed in any of claims 1-8, wherein the processor is further configured to apply a data enhancement procedure to ultrasound data for which the classifier determines a low reliability classification, and optionally wherein the data enhancement procedure comprises application of one or more filters.

10. An ultrasound data processor (22) as claimed in any of claims 1-9,
wherein the processor is adapted to derive a PWD spectrogram based on the input PWD data, and wherein the first and second analysis procedures are applied at least to a representation of the PWD spectrogram; and/or
wherein the processor is adapted to process the input PWD data to derive a PWD spectrogram based on the input PWD data, and to further extract a PWD envelope signal from the spectrogram, the envelope signal being representative of a time series of maximum Doppler frequency or velocity detected at each time point, and wherein the first and second analysis procedures are applied at least to a representation of the PWD envelope signal.

11. A system (50), comprising:

a patient monitor (54), comprising a display unit (56) and an input/output (58);
an ultrasound data processor (22) according to any of claims 1-10,
the patient monitor configured to receive from the data processor the at least one reliability classification (30) generated for the input ultrasound data, and further configured to receive an input indicative of one or more blood flow measurements (40) computed based on the same input ultrasound data, and configured to generate a display output on the display unit (56) representative of the blood flow measurements, and further representative of the reliability classification(s) for the data.

12. A system (50) according to claim 11,
wherein the ultrasound data processor (22) is a processor according to any of claims 8-10,
wherein the patient monitor (54) is arranged to receive a respective blood flow measurement (40) for each of the temporal portions of the input ultrasound signal, and
wherein the patient monitor is configured to display a trend of the blood flow measurements over time, and
the patient monitor is configured to display a visual indicator indicative of the respective reliability classification of each blood flow measurement.

13. A system (60) comprising:

an ultrasound data processor (22) as claimed in any of claims 1-10;
a wearable patch (64), the wearable patch comprising an integrated ultrasound sensing device (66) arranged to acquire ultrasound data including PWD ultrasound data;
the input/output of the ultrasound data processor being communicatively coupled to the wearable patch to receive the acquired ultrasound data from the ultrasound sensing device.

14. An ultrasound data processing method comprising:

receiving input ultrasound data, the ultrasound data including at least pulse wave Doppler (PWD) ultrasound data;
applying a first analysis procedure to the ultrasound data, or data derived therefrom, to generate a first set of one or more analysis parameters, the first analysis procedure comprising a Fourier spectral analysis of the data;
applying a second analysis procedure to the ultrasound data, or data derived therefrom, to generate a second set of one or more analysis parameters, the second analysis procedure wavelet decomposition;
inputting the first and second sets of analysis parameters to a classifier, wherein the classifier is configured to determine, based on the input analysis parameters, at least one reliability classification for the input ultrasound data indicative of reliability of at least a portion of the input ultrasound data for determining patient blood flow measurements.

15. A computer program product comprising computer program code, the computer program code being executable on a processor or computer, wherein the code is configured to cause the processor to perform a method in accordance

with claim 14.

EP 3 967 237 A1

FIG. 1

Clinically Significant Flow Values with Variations due to Irregular Heart Beats

FIG. 2

17

Beat-to-Beat Common Carotid Flow (cCO CCA)

Clinically In-significant Flow Values with Variations due to Electro-knife Noise

FIG. 3

Beat-to-Beat Common Carotid Flow (cCO CCA)

Clinically In-significant Flow Values with Variations due to Electro-knife Noise

FIG. 4

~24
Ultrasound
Data

~22
~26
I/O

~28
IC

~30
Reliability
Classification

## FIG. 5

~24
Ultrasound
Data

~34
Spectral
Analysis

~36
Wavelet
Analysis

~38
Classifier

~30
Reliability
Classification

## FIG. 6

FIG. 7

FIG. 8

FIG. 9

FIG. 10

FIG. 11

FIG. 12

FIG. 13

FIG. 14

FIG. 15

| Receive input US data | ⟋102 |
|---|---|

↓

| Apply 1st analysis procedure: spectral analysis | ⟋104 |
|---|---|

↓

| Apply second analysis procedure: wavelet analysis | ⟋106 |
|---|---|

↓

| Derive reliability classification using classifier | ⟋108 |
|---|---|

## FIG. 16

Europäisches
Patentamt

European
Patent Office

Office européen
des brevets

**EUROPEAN SEARCH REPORT**

Application Number

EP 20 19 6161

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2017/086793 A1 (SATO TAKESHI [JP]) 30 March 2017 (2017-03-30) * paragraphs [0003], [0045] * ----- | 1-15 | INV. A61B8/06 A61B8/08 G01S15/89 |
| A | US 2005/033174 A1 (MOEHRING MARK A [US] ET AL) 10 February 2005 (2005-02-10) * the whole document * ----- | 1-15 | |
| A | WO 2019/086365 A1 (KONINKLIJKE PHILIPS NV [NL]) 9 May 2019 (2019-05-09) * the whole document * ----- | 1-15 | |

TECHNICAL FIELDS
SEARCHED    (IPC)

A61B
G01S

The present search report has been drawn up for all claims

| Place of search | Date of completion of the search | Examiner |
|---|---|---|
| Munich | 23 February 2021 | Koprinarov, Ivaylo |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**ANNEX TO THE EUROPEAN SEARCH REPORT
ON EUROPEAN PATENT APPLICATION NO.**

EP 20 19 6161

This annex lists the patent family members relating to the patent documents cited in the above-mentioned European search report.
The members are as contained in the European Patent Office EDP file on
The European Patent Office is in no way liable for these particulars which are merely given for the purpose of information.

23-02-2021

| Patent document cited in search report | | | Publication date | Patent family member(s) | | | Publication date |
|---|---|---|---|---|---|---|---|
| US 2017086793 | A1 | | 30-03-2017 | JP | 6580925 | B2 | 25-09-2019 |
| | | | | JP | 2017063977 | A | 06-04-2017 |
| | | | | US | 2017086793 | A1 | 30-03-2017 |
| US 2005033174 | A1 | | 10-02-2005 | EP | 1648305 | A2 | 26-04-2006 |
| | | | | TW | 200518718 | A | 16-06-2005 |
| | | | | US | 2005033174 | A1 | 10-02-2005 |
| | | | | WO | 2005006952 | A2 | 27-01-2005 |
| WO 2019086365 | A1 | | 09-05-2019 | CN | 111432731 | A | 17-07-2020 |
| | | | | EP | 3703572 | A1 | 09-09-2020 |
| | | | | JP | 2021501656 | A | 21-01-2021 |
| | | | | US | 2020297318 | A1 | 24-09-2020 |
| | | | | WO | 2019086365 | A1 | 09-05-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **DAUBECHIES, I.** Ten Lectures on Wavelets, CBMS-NSF Regional Conference Series in Applied Mathematics. 1992
- **MALLAT, S. G.** A Theory for Multiresolution Signal Decomposition: The Wavelet Representation. *IEEE Transactions on Pattern Analysis and Machine Intelligence,* July 1989, vol. 11 (7), 674-693
- **MALLAT, S. G.** A Theory for Multiresolution Signal Decomposition: The Wavelet Representation. *IEEE Transactions on Pattern Analysis and Machine Intelligence,* July 1989, vol. 11 (7), 674-693
- **MEYER, Y. WAVELETS.** book. Cambridge University Press, 1995
- **BLANCO, P.** Volumetric blood flow measurement using Doppler ultrasound: concerns about the technique. *J Ultrasound,* 2015, vol. 18, 201-204